Europäisches Patentamt

European Patent Office

Office européen des brevets

(19)

(11) Veröffentlichungsnummer: **0 1 1 3 464**
**B1**

# (12) EUROPÄISCHE PATENTSCHRIFT

(45) Veröffentlichungstag der Patentschrift:
17.09.86

(21) Anmeldenummer: 83112650.3

(22) Anmeldetag: 15.12.83

(51) Int. Cl.⁴: **A 41 B 13/02**

(54) **Schliessband an einer Windel, insbesondere Wegwerfwindel.**

(30) Priorität: 15.12.82 JP 219828/82

(43) Veröffentlichungstag der Anmeldung:
18.07.84 Patentblatt 84/29

(45) Bekanntmachung des Hinweises auf die Patenterteilung:
17.09.86 Patentblatt 86/38

(84) Benannte Vertragsstaaten:
AT BE CH DE FR GB IT LI LU NL SE

(56) Entgegenhaltungen:
FR - A - 2 137 855
FR - A - 2 239 956
FR - A - 2 491 736
US - A - 3 926 190
US - A - 3 937 221
US - A - 3 985 136
US - A - 4 209 016

(73) Patentinhaber: UNI-CHARM CORPORATION, 182,
Shimobun Kinsei-cho, Kawanoe-shi Ehime-ken (JP)

(72) Erfinder: Suzuki, Migaku, 2666, Kawanoe-cho,
Kawanoe-shi Ehime-ken (JP)
Erfinder: Ochi, Mitsuzo, Ohaza, Fujiwara Doi-cho,
Uma-gun Ehime-ken (JP)
Erfinder: Kudo, Takeshi, 7-2-4, Yamadai Kinsei-cho,
Kawanoe-shi Ehime-ken (JP)

(74) Vertreter: Sperling, Rüdiger, Dipl.-Ing. et al,
Patentanwälte Dr.Ing.H. Finke Dipl.Ing.H. Bohr
Dipl.Ing.S. Staeger Dipl.Ing.Dipl.Wirtsch.Ing. R Sperling
Müllerstrasse 31, D-8000 München 5 (DE)

## Beschreibung

Die Erfindung betrifft ein Schliessband an einer Windel, insbesondere Wegwerfwindel, die eine obere und eine untere Lage und einen zwischen beiden Lagen angeordneten Saugkern aufweist, wobei das Schliessband einen Befestigungsbereich zum unlösbaren und einen klebfähigen Teilbereich zum lösbaren Festlegen an der Windel sowie zwischen diesen einen mit einer Trennbeschichtung versehenen Zwischenbereich minderer Steifigkeit aufweist.

Aus der US-A-3 926 190 ist ein Schliessband der genannten Gattung bekannt, das im Mittenbereich eine Querschnittsverengung aufweist. Das Schliessband ist im unbenutzten Zustand nach aussen umgefaltet und liegt auf einem Ablöseabschnitt auf, der im wesentlichen dem unlösbaren Klebbereich an der Windel entspricht. Beim Anlegen wird der freie Endabschnitt abgelöst und in seiner Längsrichtung verdreht, so dass seine Randkanten im wesentlichen spiralförmig verlaufen. Dadurch gelangt die vorher nach aussen gerichtete Klebfläche nach innen und kann somit bei angelegter Windel auf die Aussenschicht des überlappenden Aussenrandes aufgeklebt werden. Diese Ausbildung weist den Nachteil auf, dass stets eine Überlappung der Windel an den Seitenrändern erforderlich ist, was zu einem erhöhten Materialbedarf annähernd sämtlicher Komponenten führt.

Aus der US-A-3 937 221 ist ein Schliessband der genannten Gattung bekannt, bei dem der Zwischenabschnitt mit einer trennfähigen Beschichtung versehen ist. Um das freie Ende beim Anlegen von diesem Bereich abzuziehen ist ein fest auf dem lösbaren Klebbereich angebrachter Faden vorgesehen, der beim Wegziehen gleichzeitig das klebfähige Ende des Schliessbandes von der trennfähigen Beschichtung löst. Dieser Faden bleibt ständig an dem Schliessband angeklebt, um ein Lösen des Schliessbandes zum Wechseln der Windel zu ermöglichen. Auch dieses Schliessband erfordert ein Überlappen der seitlich zugeordneten Ränder der Windel, so dass auch in diesem Fall Material überflüssig ist, da das gegenseitige Überlappen nicht der eigentlichen Funktion der Windel dient. Ein solches Übermass führt zu einer Zunahme von Material sowie zu einer Bauschigkeit an den gegenüberliegenden Seiten der Teile und kann sich störend auf die Bewegungsfreiheit des Trägers auswirken.

Der Erfindung liegt die Aufgabe zugrunde, bei einer Windel der genannten Gattung ein Schliessband zu schaffen, bei dem ohne Einschränkung der Funktion und des Tragekomforts die Länge der Taille bei einer Windel ohne überlappende Randbereiche der einander gegenüberliegenden Seitenränder einstellbar ist.

Die Aufgabe wird erfindungsgemäss dadurch gelöst, dass

a) das Schliessband als Grundmaterial auf der dem Körper des Trägers zugewandten Seite ein Faservlies aufweist, wobei

b) der sich an den Zwischenbereich nach aussen anschliessende Bereich eine hohe Steifigkeit aufweist und am Ende an den klebfähigen Teilbereich angrenzend einen nicht klebenden Griffbereich besitzt.

Durch die angegebenen Massnahmen ergibt sich für den Träger ein angenehmes Tragegefühl, da die Haut stets mit einem weichen Faservlies in Berührung kommt, so dass ein Kratzen oder Scheuern vermieden wird.

Die hohe Steifigkeit des zweiten Bereichs kann günstigerweise dadurch erreicht werden, dass ein Kunststofffilm integral in das Faservlies eingeschmolzen wird.

Bei einem bevorzugten Ausführungsbeispiel ist das Schliessband 20 bis 80 mm breit und erstreckt sich von den Aussenrändern des bezeichneten hinteren Bereichs über eine Länge von 70 bis 130 mm nach aussen, wobei der brückenartige Zwischenbereich, der klebfähige Teilbereich und der Griffbereich jeweils eine Länge von 40 bis 80 mm, 20 bis 40 mm und 5 bis 15 mm aufweisen.

Die niedrige Steifigkeit beträgt 0,27 bis 33,8 des Werts gemäss den Vorschriften des JIS (Japanese Industrial Standard) P 8143-1967, während die hohe Steifigkeit zwischen 2.16 bis 156 dieses Wertes beträgt.

Im weiteren werden anhand der beigefügten Zeichnungen verschiedene Ausführungsbeispiele der Erfindung näher erläutert.

Es zeigen:

Fig. 1 eine Ansicht einer Abwicklung einer erfindungsgemässen Wegwerfwindel mit Schliessband mit teilweise herausgebrochenem Bereich,

Fig. 2 eine perspektivische Ansicht der Wegwerfwindel aus Fig. 1 im angelegten Zustand,

Fig. 3 eine Teilansicht eines vergrösserten Bereichs der Windel aus Fig. 1, in der der Bereich dargestellt ist, in dem das Schliessband an der Windel befestigt ist,

Fig. 4 eine schematische Darstellung eines Schnitts entlang der Linie 4-4 in Fig. 3,

Fig. 5 eine schematische Darstellung im vergrösserten Massstab eines Schnitts entlang der Linie 5-5 in Fig. 3,

Fig. 6 eine Draufsicht auf ein Ausführungsbeispiel eines abgewickelten Schliessbands gemäss der vorliegenden Erfindung,

Fig. 7A eine Teilansicht im vergrösserten Massstab eines weiteren Ausführungsbeispiels eines erfindungsgemäss ausgebildeten Schliessbands bevor die Komponentenmaterialien miteinander laminiert werden, um den Bereich hoher Festigkeit zu bilden,

Fig. 7B eine Ansicht ähnlich derjenigen in Fig. 7A nachdem die Materialkomponenten laminiert worden sind,

Fig. 8A eine teilweise perspektivische Ansicht eines erfindungsgemässen Schliessbands, das im Klebbereich mit Schlitzen versehen ist,

Fig. 8B eine Ansicht ähnlich derjenigen in Fig. 8A, wobei jedoch das erfindungsgemässe Schliessband im nichtklebenden Bereich nicht mit Schlitzen versehen ist,

Fig. 8C eine Ansicht ähnlich derjenigen in Fig. 8A, wobei das erfindungsgemässe Schliessband in dem brückenartigen Zwischenbereich als auch in dem Klebbereich mit den Schlitzen versehen ist,

Fig. 9A eine teilweise perspektivische Ansicht eines erfindungsgemässen Schliessbandes, bei welchem der Klebbereich eine Vielzahl von intermittierend angeordneten Klebstreifen aufweist, und

Fig. 9B eine Ansicht ähnlich wie in Fig. 9A eine andere Ausführungsform darstellend.

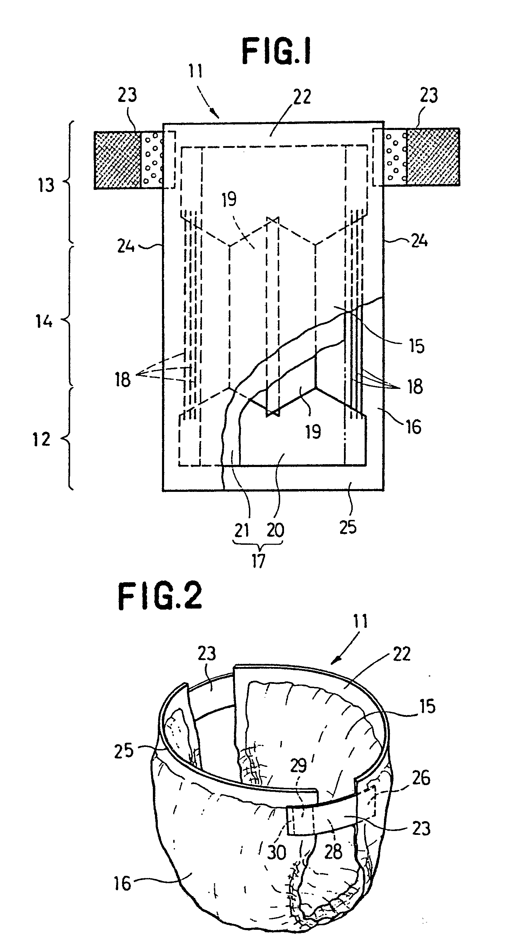

Die Figuren 1 und 2 zeigen ein Ausführungsbeispiel einer erfindungsgemässen Wegwerfwindel 11 mit einem Schliessband 23. Die Wegwerfwindel 11 weist einen vorderen Bereich 12, einen hinteren Bereich 13 und einen Schrittbereich 14 auf und besteht aus einer wasserdurchlässigen oberen Lage 15 aus Faservlies oder ähnlichem als Grundmaterial, einer wasserundurchlässigen unteren Lage 16 aus Plastikfilm oder ähnlichem, einem zwischen den beiden genannten Lagen 15 und 16 angeordneten Saugkern 17 aus flockigem Pulpenmaterial oder ähnlichem und elastischen Teilen 18, die sich in Längsrichtung an den beiden sich gegenüberliegenden Seiten des zwischen den beiden Lagen 15 und 16 angeordneten Saugkerns 17 erstrecken.

Der Saugkern 17 besteht aus einem ersten Saugteil 20 mit nach innen in den Schrittbereich 14 gefalteten Seitenklappen 19 und einem zweiten Saugteil 21, das breiter ist als der Schrittbereich 14, in dem das erste Teil 20 hineingefaltet ist, jedoch schmäler als die Bereiche des Teils 20 im vorderen und im hinteren Bereich 12, 13.

Die elastischen Teile 18 bestehen jeweils aus einer Vielzahl von Gummifäden, die mittels eines heissschmelzenden elastischen Klebmittels bekannter Art in Querabständen an der Winkel 11 festgeklebt sind. Diese Gummistränge können zwischen 2 bis 45 Fäden mit Ausdehnungsprozentsätzen von 100 bis 400% aufweisen, wobei deren Querschnitt zwischen 0,03 und 0,45 mm² und der Gesamtquerschnitt zwischen 0,06 und 1,35 mm² liegen kann. Obwohl elastische Teile 18 der beschriebenen Art bevorzugt werden, kann ein relativ schmales einzelnes Gummiband oder ein relativ breiter Streifen aus Polyurethan-Schaum, welche beide in üblicher Weise bei Wegwerfwindeln dieses Typs in Gebrauch sind, verwendet werden.

Mit dem Bezugszeichen 22 ist im hinteren Bereich 13 der Wegwerfwindel 11 eine Taille definiert, die an jedem Seitenrand mit einem Schliessband 23 ausgestattet ist.

Das Schliessband 23 erstreckt sich um 70 bis 130 mm über die zugeordneten seitlichen Enden oder Ränder 24 der Wegwerfwindel 11 hinaus. Es ist erkennbar, dass das Schliessband 23 beträchtlich länger ist als Klebebänder, die üblicherweise an Wegwerfwindeln dieser Art verwendet werden. Diese Ausbildung des Schliessbands 23 ermöglicht eine geringere Breite der Wegwerfwindel 11 als bei üblichen Wegwerfwindeln, so dass diese Windel 11, wenn sie dem Benutzer angelegt ist, zwischen dem rückwärtigen Taillenbereich und dem vorderen Taillenbereich an den einander gegenüberliegenden Seitenrändern einen freien Spalt bildet, der von den jeweiligen Schliessbändern 23 überbrückt wird, so dass die sich überlappenden Bereiche, die bei der konventionellen Windel entstehen, durch diesen Überbrückungsbereich ersetzt werden.

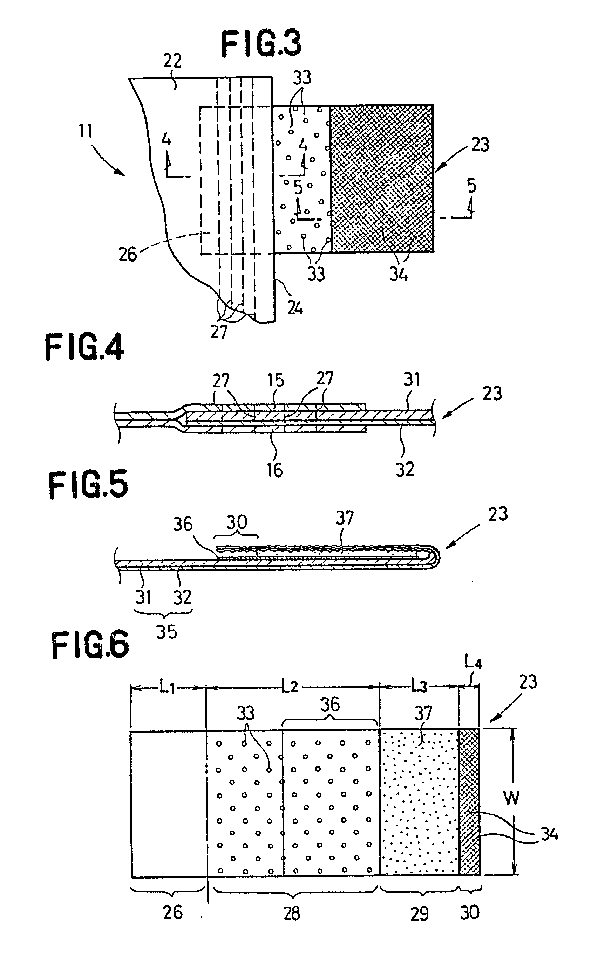

Die Figuren 3 bis 6 zeigen das Schliessband 23 näher im Detail. Das Schliessband 23 ist durch Einstecken eines Befestigungsbereichs 26 zwischen die obere Lage 15 und die untere Lage 16 und integrales Verschweissen dieses Befestigungsbereichs 26 mit diesen beiden Lagen an der Windel 11 befestigt. Eine derartige Befestigung kann auch durch Kleben erzielt werden; beim Einschweissen wird vorzugsweise eine Überschallverschweissung entlang der in Fig. 3 dargestellten unterbrochenen Linien 27 durchgeführt.

Das üblicherweise für die bekannten Windeln praktisch verwendete Klebband ist stets an der Aussenfläche der unteren Lage 11 festgeklebt, mit der Folge, dass ein Teil der unteren Lage, der diesen Befestigungsbereich 26 einschliesst, neben diesem Befestigungsbereich während des Gebrauchs kräftig unter Zug steht und gedehnt wird, wodurch die ursprüngliche Festigkeit dieser unteren Lage stark reduziert wird, oder diese Lage selber beschädigt wird. Um diesen Bereich neben dem Befestigungsbereich zu verstärken, werden bereits verschiedene Massnahmen vorgeschlagen. Beispelsweise ist in dem US-Patent 3 867 940 der Bereich der unteren Lage, an welchem das Klebband befestigt ist, an der Innenseite mit einem Stück Baumwollstoff versehen; in US-Patent 4 055 182 ist offenbart, dass der Befestigungsbereich des Klebebands selber auf der Innenfläche mit einer heissschmelzenden Lage beschichtet wird, und aus dem US-Patent 3 848 594 ist bekannt, das Klebeband und das gegossene Ablöseband gemeinsam an der Windel zu befestigen, so dass beide Bänder am Aussenende der Windel eine Y-Form ausbilden.

Demgegenüber ermöglicht das erfindungsgemässe Schliessband 23 den Befestigungsbereich 26 in zufriedenstellender Weise an der Windel festzulegen, und es gestattet dem Bereich neben dem Befestigungsbereich einschliesslich dieses Befestigungsbereichs selber, eine adäquate Zugfestigkeit zu bewahren.

Das Schliessband 23 weist von einem Ende zum anderen nacheinander den Befestigungsbereich 26, einen brückenartigen Zwischenbereich 28, einen klebfähigen Teilbereich 29 und einen Griffbereich 30 auf. Vorzugsweise besitzt der Befestigungsbereich eine Länge $L_1$ von mindestens 15 mm, der brückenartige Zwischenbereich 28 eine Länge $L_2$ von 40 mm bis 80 mm, der klebfähige Teilbereich 29 eine Länge $L_3$ von 20 mm bis 40 mm, der Griffbereich 30 eine Länge $L_4$ von 5 mm bis 15 mm und das gesamte Schliessband 23 eine Breite W von 20 bis 80 mm.

Das Schliessband besteht aus Laminatlagen aus einem Faservlies 31 und einem Plastik- oder Kunststofffilm 32 als Basismaterial. Die Laminatlagen werden teilweise miteinander verschmolzen (wie beispielsweise mit der Bezugsziffer 33 gekennzeichnet), so dass dieses Material eine relativ niedrige Steifigkeit in dem brückenartigen Zwischenbereich 28 aufweist.

Die Laminatlagen können vollkommen miteinander verschweisst werden ohne dass die Steifigkeit zunimmt. In dem klebfähigen Teilbereich 29 und in dem Griffbereich 30 sind diese laminierten Lagen nicht nur miteinander verschweisst, sondern dicht miteinander bossiert (vgl. Bezugsziffer 34), um eine relativ hohe Steifigkeit zu erzielen. Eine derartige thermische Prägeverbindung bewirkt, dass der Kunststoffilm 32 zumindest teilweise schmilzt und dabei in die freien Räume zwischen den Fasern des Faservlieses eintritt und integraler Bestandteil dieses Faservlieses 31 wird. Als Ergebnis erhält man im klebfähigen Teilbereich 29 und im Griffbereich 30 die gewünschte Steifigkeit, die nachfolgend näher im Detail beschrieben wird; die Zwischenräume zwischen den Fasern des Faservlieses 31 sind ausgefüllt, damit das Aufbringen von Klebmaterial erleichtert wird, was ebenfalls nachfolgend näher im Detail beschrieben wird; desweiteren wird die Festigkeit des Faservlieses erhöht.

Um das Grundmaterial 35 des Schliessbandes 23 mit dem klebfähigen Teilbereich 29 und dem Griffbereich 30 zu erhalten, wird ein Faservlies 31 verwendet mit einem Gewicht von 20 bis 30 g/m² und einer Dichte von 0,1 bis 0,3 g/cm³, das aus Polyester, Nylon, Polypropylen, Rayonfasern oder ähnlichem oder einer Mischung aus den genannten Materialien besteht, während der Kunststofffilm 39 eine Dicke von 20 bis 60 µm und eine Dichte von 0,91 bis 0,97 g/cm³ aufweist und aus einem Material besteht, dessen Schmelzpunkt geringer ist als derjenige der Fasern, beispielsweise Polyäthylen, Polypropylen oder Polyurethan, einschliesslich Schaum; derartige Materialien werden bei dem Ausbildungsbeispiel bevorzugt. Das Faservlies 31 und der Film 32 können aufeinandergelegt und unter Druck in einer entsprechenden Schmelz- oder Prägeform miteinander verbunden werden, wobei die Temperatur auf einen solchen Wert gebracht wird, der nahe am Schmelzpunkt dieses Films liegt. Um einen gewünschten integrierten Zustand ohne Bossieren im Grundmaterial 35 zu erhalten, können die Teilbereiche des Grundmaterials mittels eines heissen Flachrollers zusammengepresst werden.

Das auf diese Weise erhaltene Grundmaterial wird an der Oberfläche des Faservlieses 31 im brückenartigen Zwischenbereich 28 mit einem bekannten Ablöseagenz, beispielsweise Silicon, beschichtet, um eine Ablöselage 36 zu bilden; desweiteren wird die Oberfläche des Faservlieses im Teilbereich 29 mit einem druckempfänglichen Klebestoff beschichtet, um die Klebelage 37 mit einem Gewichtswert von 30 bis 160 g/m² zu bilden. Die auf das Faservlies 31 aufgebrachte Menge des Ablöseagenz für die Ablöselage 36 wird so eingestellt, dass eine gewünschte Ablösefunktion erzielt wird, ohne die Steifigkeit des Faservlieses 31 zu erhöhen. Der brückenartige Zwischenbereich 28 kann vollständig mit dem Ablöseagenz beschichtet werden, wobei die Beschichtung mit einer Dicke von weniger als 20 µm durchgeführt wird. Darüberhinaus ist es nicht erforderlich, die Ablöselage 36 als eine kontinuierliche Lage auszubilden, soweit diese Lage 36 eine gewünschte Ablösefähigkeit besitzt. Statt dessen kann das Faservlies 31 nur bereichsweise an den Fasern mit dem Ablösemittel versehen sein oder teilweise freigelegt sein.

Das auf diese Weise gebildete Schliessband 23 weist im brückenartigen Zwischenbereich 28 eine Steifigkeit von 0,27 bis 33,8, der klebfähige Teilbereich 29 und der Griffbereich 30 eine Steifigkeit von 2,16 bis 156 des Werts auf, wie er nach dem Verfahren A gemäss des JIS (Japanese Industrial Standard) P 8143-1967 gemessen wird. Der brückenartige Zwischenbereich 28 hat eine Zugfestigkeit von 4 kg/50 mm oder höher, diese Zugfestigkeit wird durch eine Messung erhalten, bei der ein Sample von 50 mm mit einer Zugspannung von 30 cm/min in einem Spannungstester gedehnt wird.

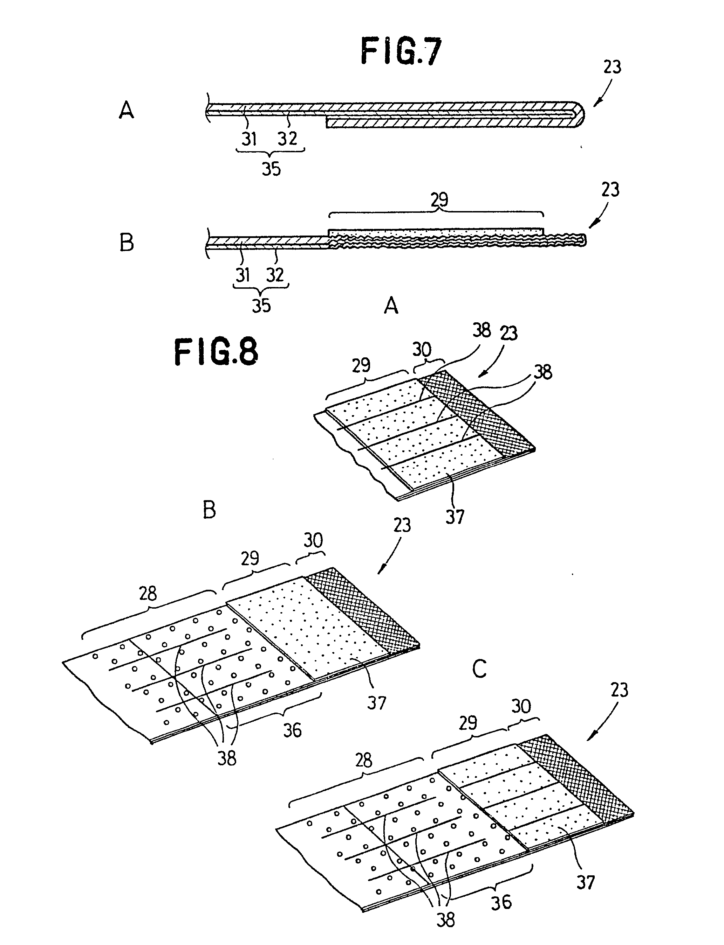

In den Figuren 7A und 7B ist ein weiteres Ausführungsbeispiel des Aufbaus eines klebfähigen Teilbereichs 29 und eines Griffbereichs 30 dargestellt. Bei dieser Ausführungsform wird ein Ende des Grundmaterials 35 umgefaltet, so wie es in Fig. 7A dargestellt ist, um den Plastik- oder Kunststofffilm 32 mit sich selber in Kontakt zu bringen; dann wird das vorher beschriebene Verfahren des Bossierens angewendet, wobei der besagte Plastik- oder Kunststofffilm 32 in das Faservlies 31 an dessen Ober- und Unterseite integral einschmolzen wird (vgl. Fig. 7B). Auf diese Weise kann dem klebfähigen Teilbereich 29 und dem Griffbereich 30 die gewünschte Steifigkeit verliehen werden, selbst wenn das Faservlies 31 ein geringes Flächengewicht und eine niedrige Dichte besitzt und ein Plastik- oder Kunststofffilm von extrem geringer Stärke und niedriger Dichte verwendet wird. Der im wesentlichen gleiche Effekt, der durch diese Art des Aufbaus erzielt wird, kann auch erreicht werden, wenn der Kunststofffilm 32 sandwichartig zwischen zwei Lagen des Faservlieses 31 eingelagert wird; dies ist in der Zeichnung nicht dargestellt. In einem solchen Fall können die einzelnen Komponenten ebenfalls miteinander nahezu verschweisst werden, ohne die Anordnung einem Bossierverfahren zu unterziehen.

In dem auf diese Weise hergestellten Schliessband 23 wird das äussere Ende zurückgefaltet, wenn es nicht verwendet wird, so dass die Klebeschicht 37 vorläufig an der Ablöselage 36 befestigt ist, so wie es in Fig. 5 dargestellt ist; der Griffbereich 30 kann mit den Fingern angefasst und bei Bedarf von der Ablöselage 36 abgezogen werden (vgl. Fig. 6).

Wenn die Windel 11 dem Benutzer angelegt wird, wird der klebfähige Teilbereich 29 des Schliessbandes 23 an der unteren Lage 16 angeklebt, so dass sich der brückenartige Zwischenbereich 28 zwischen dem Befestigungsbereich 26 an der Windel und dem klebfähigen Teilbereich 29 erstreckt und in jede Richtung verformbar ist und dabei in weichem Kontakt mit dem Körper des Benutzers steht, da er nicht nur eine niedrige Steifigkeit (oder hohe Flexibilität) sondern auch eine mehr oder weniger hohe Elastizität besitzt und das Faservlies so angeordnet ist, dass es den Körper des Trägers berührt (vgl. Fig. 4 und 5).

Das Schliessband 23 ist auch geeignet, die Taillenweite an die individuellen Träger anzupassen, da der brückenartige Zwischenbereich 28 eine entsprechende Länge für ein derartiges Einstellen aufweist. Die Tatsache, dass der klebfähige Teilbereich 29 und der Griffbereich 30 des Schliessbandes 23 eine entsprechende hohe Steifigkeit besitzen, erleichtert nicht nur das Befestigen und die Abziehoperation hinsichtlich der unteren Lage 16, sondern sichert auch die Befestigungswirkung des bezeichneten klebfähigen Teilbereichs 29 hinsichtlich der unteren Lage 16. Eine derartige verlässliche Befestigung wird darüberhinaus durch die Tatsache sichergestellt, dass der brückenartige Zwischenbereich 28 eine Steifigkeit besitzt, die geringer ist als diejenige des klebfähigen Teilbereich 29, so dass eine Kraft, die aufgrund einer Bewegung des Trägers auf den brückenartigen Zwischenbereich 28 ausgeübt wird, von diesem reduziert oder absorbiert wird. Darüberhinaus weist der Griffbereich 30, wenn er bossiert ist, eine rauhe Oberfläche auf, die ein Abrutschen der Finger während des Befestigens oder Abziehens verhindert. Derartige Handgriffe werden auf dise Weise weiter erleichtert.

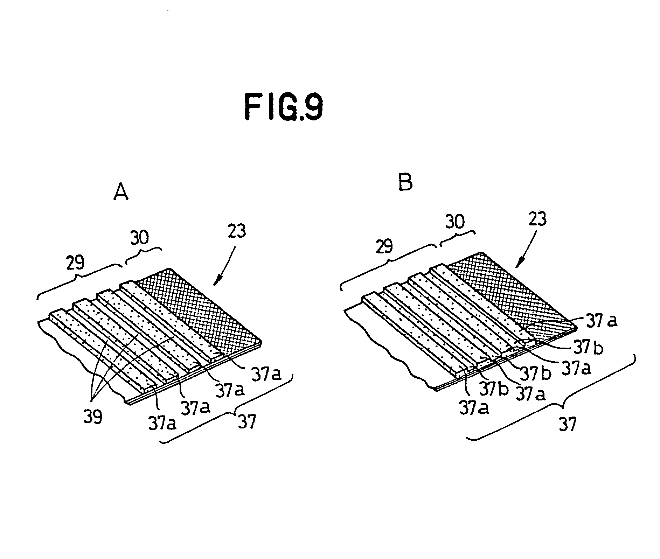

In den Figuren 8A, 8B und 8C sind jeweils Ausführungsbeispiele dargestellt, bei denen eine Vielzahl von Schlitzen 38 in Querrichtung in dem Schliessband 23 je nach Ausführungsbeispiel jeweils im klebfähigen Teilbereich 29, im brückenartigen Zwischenbereich 28 oder in beiden, dem brückenartigen Zwischenbereich und dem klebfähigen Teilbereich vorgesehen sind; diese Schlitze 38 erstrecken sich durch die jeweiligen Bereiche von der Oberfläche bis zur unteren Fläche. Diese Schlitze 38 können durch einen einzigen zentralen Schlitz ersetzt werden, sie können intermittierend oder kontinuierlich sein.

Wenn die Windel einem Träger angelegt wird, kann festgestellt werden, dass das Schliessband 23 verschiedenen Kräften ausgesetzt ist, die während einer Bewegung des Trägers auftreten; diese Kräfte können Schwerkräfte sein, Abziehkräfte oder Verformungskräfte, so dass befürchtet werden kann, dass der klebfähige Teilbereich 29 von der unteren Lage abgezogen werden könnte, wenn derartige verschiedene Kräfte ein gewisses kritisches Mass erreichen.

Das Vorsehen der bezeichneten Schlitze 38 in dem brückenartigen Zwischenbereich 28 und/oder dem klebfähigen Teilbereich 29 gestattet es den jeweiligen Abschnitten dieser Bereiche, die von den Schlitzen 38 gebildet werden, unabhängig voneinander zu funktionieren und daher können die Kräfte, die auf den brückenartigen Zwischenbereich 28 und den bezeichneten klebfähigen Bereich 29 aufgebracht werden, wirkungsvoll absorbiert oder verteilt werden.

In den Figuren 9A und 9B ist eine Ausführungsvariante des klebfähigen Teilbereichs 29 dargestellt. In dieser Variante ist der klebfähige Teilbereich 29 so ausgebildet, dass in Längsrichtung des Schliessbandes 23 intermittierend Rippen 37 mit Kleblage und Nuten 39 aufweist, in welchen keine Kleblage vorhanden ist, wobei diese verschiedenen Ausbildungen abwechselnd angeordnet sind. Diese alternierend ausgebildeten Rippen und Nuten sind in Fig. 9 mit den Bezugsziffern 37a und 37b bezeichnet. Abhängig von den Abmessungen des klebfähigen Teilbereichs 29 beträgt die Breite jeder Rippe 37a zwischen 3 mm bis 10 mm und die jeder Nut 37b oder 39 zwischen 1 mm und 5 mm.

Die gesamte Fläche, die von den Nuten 37a oder 39 bedeckt wird, beträgt weniger als 50, vorzugsweise zwischen 10% bis 30% des gesamten klebfähigen Teilbereichs 29.

Üblicherweise hat ein flexibler Plastikfilm aus Polyäthylen oder ähnlichem, der auf die untere Lage 16 der Windel aufgeklebt worden ist, diese untere Lage oft gedehnt oder sogar beschädigt, wenn der klebfähige Teilbereich 29 des Schliessbandes 23 von dieser unteren Lage 16 abgezogen worden ist, um nachzuprüfen, ob die Windel bereits mit Inhalt versehen worden ist oder nicht, oder wenn die Windel an der Taille nachangepasst werden sollte.

Gemäss der vorliegenden Erfindung werden derartige Nachteile vermieden, da die Oberfläche der klebfähigen Lage 37, die an der unteren Lage 16 angeklebt wird, in Längsrichtung des Schliessbandes 23, wie vorher beschrieben, intermittierend ausgebildet ist.

## Patentansprüche

1. Schliessband an einer Windel, insbesondere Wegwerfwindel, die eine obere und eine untere Lage (15, 16) und einen zwischen beiden Lagen angeordneten Saugkern (17) aufweist, wobei das Schliessband einen Befestigungsbereich (26) zum unlösbaren und einen klebfähigen Teilbereich (29) zum lösbaren Festlegen an der Winkel sowie zwischen diesen einen mit einer Trennbeschichtung (36) versehenen Zwischenbereich (28) minderer Steifigkeit aufweist, dadurch gekennzeichnet, dass
a) das Schliessband als Grundmaterial auf der dem Körper des Trägers zugewandten Seite ein Faservlies (31) aufweist, wobei
b) der sich an den Zwischenbereich (28) nach aussen anschliessende Bereich eine hohe Steifigkeit aufweist und am Ende an den klebfähigen Teilbereich (29) angrenzend einen nicht klebenden Griffbereich (30) besitzt.

2. Schliessband nach Anspruch 1, dadurch gekennzeichnet, dass die hohe Steifigkeit des zweiten Bereichs dadurch erreichbar ist, dass ein Kunststofffilm (32) integral in das Faservlies (31) eingeschmolzen ist.

3. Schliessband nach Anspruch 1, dadurch gekennzeichnet, dass der klebfähige Teilbereich (29) und der Griffbereich (30) bossiert sind.

4. Schliessband nach mindestens einem der Ansprüche 1-3, gekennzeichnet durch eine Breite zwischen 20 mm und 80 mm und eine Erstreckung über die zugeordneten Seitenränder des hinteren Bereichs hinaus von 70 mm-130 mm, wobei der brückenartige Zwischenbereich (28) ca. 40 mm-80 mm, der klebfähige Teilbereich ca. 20 mm-40 mm und der Griffbereich zwischen ca. 5 mm und 15 mm lang sind.

5. Schliessband nach mindestens einem der Ansprüche 1-4, dadurch gekennzeichnet, dass die niedrige Steifigkeit zwischen 0,27-33,8 des Wertes nach den Vorschriften des JIS (Japanese Industrial Standard) — P 8143-1967 beträgt, während die höchste Steifigkeit 2,16-156 dieses Wertes beträgt.

6. Schliessband nach mindestens einem der Ansprüche 1-5, dadurch gekennzeichnet, dass in Längsrichtung mit Ausnahme des Griffbereichs (30) eine gewünschte Anzahl von Schlitzen in dem Band vorgesehen sind.

7. Schliessband nach mindestens einem der Ansprüche 1-6, dadurch gekennzeichnet, dass der klebfähige Teilbereich auf der oberen Fläche vorgesehen ist und zur unteren Lage gewandt ist, wobei Kleblagen in Längsrichtung intermittierend auf dem Schliessband (23) vorgesehen sind.

8. Schliessband nach mindestens einem der Ansprüche 1-7, dadurch gekennzeichnet, dass die Verbindung des Schliessbands mit jeder Seite des hinteren Bereichs der Windel durch integrales Anhaften eines Endes des Bandes zwischen der oberen und der unteren Lage (15, 16) stattfindet.

**Claims**

1. A waist band provided on a diaper, in particular on a disposable diaper, including a topsheet and a backsheet (15, 16) as well as an absorbent core (17) interposed between said two sheets, said waist band being provided with an attachment area (26) for non-releasable fastening to the diaper and with an adhesive sub-area (29) for releasable fastening to the diaper, an intermediate area (28) of reduced rigidity being disposed between said attachment area and said adhesive sub-area and being provided with a separator layer (36), characterized in that
a) the waist band is provided with a nonwoven fabric (31) as a basic material on the side facing the user's body,
b) the area following said intermediate area (28) towards the outward end having a high rigidity and being provided with a non-adhesive handling area (30) arranged at the end thereof adjacent the adhesive sub-area (29).

2. A waist band according to claim 1, characterized in that the high rigidity of said second area is achieved by melting a plastic film (32) into said nonwoven fabric (31) so as to be integrated therewith.

3. A waist band according to claim 1, characterized in that the adesive sub-area (29) and said handling area (30) are embossed.

4. A waist band according to at least one of the claims 1 to 3, characterized in that it has a width of 20 to 80 mm and that it extends beyond the associated side ends of the rear area by a length of 70 to 130 mm, said bridge-like intermediate area (28) being approx. 40 to 80 mm long, said adhesive sub-area being approx. 20 to 40 mm long and said handling area being approx. 5 to 15 mm long.

5. A waist band according to at least one of the claims 1 to 4, characterized in that the low rigidity is 0.27 to 33.8 at the value according to the provisions of JIS (Japanese Industrial Standard) — p 8143-1967, whereas the highest rigidity is 2.16 to 156 at said value.

6. A waist band according to at least one of the claims 1 to 5, characterized in that said waist band is longitudinally provided, with the exception of said handling area (30), with a desired number of slots.

7. A waist band according to at least one of the claims 1 to 6, characterized in that the adhesive sub-area is provided on the upper surface and faces said backsheet, adhesive layers being intermittently arranged on said waist band (23) in the longitudinal direction.

8. A waist band according to at least one of the claims 1 to 7, characterized in that connection of said waist band to each side of the rear area of the diaper is achieved by integrally adhering one end of said band between said topsheet and said backsheet (15, 16).

**Revendications**

1. Bande de fermeture d'une couche, en particulier d'une couche à jeter, qui possède une couche supérieure et une couche inférieure (15, 16) et un noyau absorbant (17) disposé entre les deux couches, la bande de fermeture présentant une zone de fixation (26) destinée à une fixation inamovible à la couche, et une zone partielle adhésive (29) destinée à une fixation amovible à la couche, et, entre ces zones, une zone intermédiaire (28), de rigidité plus faible, pourvue d'un revêtement de séparation (36), caractérisée en ce que
a) la bande de fermeture présente, en tant que matière de base, un non-tissé (31) sur le côté dirigé vers le corps du porteur,
b) la zone immédiatement contiguë à la zone intermédiaire (28) et dirigé vers l'extérieur présentant une rigidité élevée et possédant, contiguë à l'extrémité de la zone partielle adhésive (29), une zone de saisie (30), non-adhésive.

2. Bande de fermeture selon la revendication 1, caractérisée en ce qu'on peut arriver à la rigidité élevée de la deuxième zone en incorporant dans le non-tissé (31), par fusion, un film plastique (32) qui en fasse partie intégrante.

3. Bande de fermeture selon la revendication 1, caractérisée en ce que la zone partielle adhésive (29) et la zone de saisie (30) sont gaufrées.

4. Bande de fermeture selon au moins l'une des revendications 1 à 3, caractérisée par une largeur comprise entre 20 mm et 80 mm, et une extension de 70-130 mm au-delà des bords lateraux correspondants de la zone arrière, la zone partielle en pont (28) ayant une longueur d'environ 40 mm-80 mm, la zone partielle adhésive une longueur d'environ 20 mm-40 mm et la zone de saisie une longueur comprise entre environ 5 mm et 15 mm.

5. Bande de fermeture selon au moins l'une des revendications 1 à 4, caractérisée en ce que la faible rigidité est comprise entre 0,27-33,8 fois la valeur selon les prescriptions de JIS (Norme Industrielle Japonaise) P 8143-1967, tandis que la rigidité la plus élevée est de 2,16-156 fois cette valeur.

6. Bande de fermeture selon au moins l'une des revendications 1 à 5, caractérisée en ce que, dans le sens longitudinal, à l'exception de la zone de saisie (30), il est prévu un nombre quelconque voulu de fentes dans la bande.

7. Bande de fermeture selon au moins l'une des revendications 1 à 6, caractérisée en ce que la zone partielle adhésive est prévue sur la surface supérieure et est dirigée vers la couche inférieure, des couches adhésives étant prévues, d'une manière intermittente sur la bande de fermeture (23), dans le sens longitudinal.

8. Bande de fermeture selon au moins l'une des revendications 1 à 7, caractérisée en ce que la liaison de la bande de fermeture avec chaque côté de la zone arrière de la couche est réalisée par adhérence intégrale d'une extrémité de la bande entre le couche supérieure et la couche inférieure (15, 16).

# FIG.1

# FIG.2

# FIG.3

# FIG.4

# FIG.5

# FIG.6

# FIG.7

A

31 32
35

23

B

29

31 32
35

23

A

# FIG.8

29 30 38 23

38

37

23

B

28 29 30

23

38

37

36

C

29 30 23

28

38

37

36

# FIG.9

A

B